# EUROPEAN PATENT APPLICATION

(11) **EP 0 690 129 A1**
(43) Date of publication of application: **03.01.1996**
(21) Application number: 95111593.0
(22) Date of filing: 29.08.1990
(51) Int. Cl.: C12N 15/85, C12N 15/52, C12N 15/86, C12N 7/01, A61K 48/00, A61K 31/70, A61K 45/06

(54) **Novel entities for cancer therapy**

(30) Priority: 30.08.1989 GB 8919607
(62) Divisional of application: 90309430.8
(71) Applicant: THE WELLCOME FOUNDATION LIMITED, London NW1 2BP (GB)
(72) Inventor: Huber, Brian, Durham, North Carolina 27713 (US); Richards, Cynthia Ann, Durham, North Carolina 27713 (US); Krenitsky, Thomas Anthony, Chapel Hill, North Carolina 27514 (US)
(74) Representative: Stott, Michael John

(57) **Abstract**

Novel molecular chimaeras produced by recombinant DNA techniques are described. They comprise a target tissue specific transcriptional regulatory sequence (TRS) linked and controlling the expression of a heterologous enzyme for example Varicella Zoster Virus Thymidine Kinase (VZV TK) gene. A molecular chimaera is packaged into a synthetic retroviral particle which is capable of infecting mammalian tissue. This in turn may be administered to a patient, and the TRS will be selectively transcriptionally activated in the target tissue (for example cancerous tissue). Administration of compounds which are selectively metabolised by the enzyme produce cytotoxic or cytostatic metabolites in situ thereby selectively killing or arresting the growth of the target cell.

## Description

The present invention relates to molecular chimaeras, infective virions, to methods of their construction, to pharmaceutical formulations containing them, to their use in therapy, particularly virus-directed enzyme prodrug therapy, particularly in the treatment of cancers, more particularly in the treatment of hepatocellular carcinoma, and to the use of agents which can be catalysed by a heterologous enzyme to cytotoxic or cytostatic metabolites, such as purine arabinosides and substituted pyrimidines in virus-directed enzyme prodrug therapy.

Cancer of all forms is one of the major causes of morbidity throughout the world. Research in the area of cancer chemotherapy has produced a variety of antitumour agents which have differing degrees of efficacy. Standard clinically used agents include adriamycin, actinomycin D, methotrexate, 5-fluorouracil, cis platinium, vincristine and vinblastine. However, these presently available antitumour agents are known to have various disadvantages such as toxicity to healthy cells and resistance of certain tumour types. Other forms of therapy such as surgery, are known. However it is appreciated by those skilled in the art that novel approaches and entities for cancer therapies are required.

Hepatocellular carcinoma (HCC) is one of the major malignant diseases in the world today; the greatest incidence being in Japan, China, other parts of the Asia and sub-Saharan Africa. Recent evidence suggests that the incidence of hepatocellular carcinoma in Europe and North America is increasing. The disease is estimated to be responsible for or involved in up to approximately 1,250,000 deaths a year and as such is numerically, one of the world's major malignant diseases.

The prognosis of HCC is always poor with the world-wide frequency rate almost equalling the mortality rate. After diagnosis, the median survival time is less than four months. Long-term survival, defined as survival longer than one year after diagnosis, is seen only occasionally. Most HCC patients succumb to either the complications of liver failure with or without massive bleeding, or to the general effects of a large tumour burden, with cachexia, malnutrition, infection and sepsis. Though distant metastases occur (up to 90% of patients have metastatic tumour at time of death), regional disease most often limits survival. Consequently, therapies directed towards control of hepatic tumour are appropriate although it will be appreciated that treatment of the metastatic disease is also of great importance. (Kew M.C. Postgraduate Medical Journal 59 (Suppl.) 78-87 (1983) and Berk.P. (Ed) Seminars in Liver Disease 4, No.2, Thieme-Stratton Inc. N.Y. N.Y. (1984)).

Current therapies available to the clinician are on the whole ineffective as a cure for this disease (Nerenstone et al, Cancer Treatment Reviews 15, 1-31 (1988)). To date, surgery continues to be the only potential cure. However, at the time of diagnosis, the overwhelming majority of patients are not able to undergo radical surgery. In certain studies (Nerenstone et al supra) it was found that less than 3% of patients were considered capable of undergoing surgery and of the small percentage that so do approximately 50% suffer from postoperative morbidity (Nerenstone et al supra).

Systemic single and combination agent chemotherapy and radiation are relatively ineffective and this emphasises the need for new approaches and therapies for the treatment of this disease.

Gene therapy involves the stable integration of new genes into particular target cells and the expression of those genes, once they are in place, to alter the phenotype of that particular target cell (for review see Anderson,W.F. Science 226: 401-409, 1984; McCormick, D. Biotechnology 3: 690-693, 1985) . Gene therapy may be beneficial for the treatment of genetic disease which involve the replacement of one defective or missing enzyme, such as hypoxanthine-guanine phosphoribosyl transferase in Lesch Nyhan disease; purine nucleoside phosphorylase in severe immunodeficiency disease and adenosine deaminase in severed combined immunodeficiency disease. As yet, gene therapy has not been used in the clinic, although in certain disorders, certain types of gene therapy are likely to be used imminently.

It has been found by the present inventors that it is possible to selectively arrest the growth of or kill mammalian carcinoma cells with chemical agents capable of selective conversion to cytotoxic or cytostatic metabolites. This is achieved by the construction of a molecular chimaera comprising a "target tissue-specific" transcriptional regulatory sequence (TRS) which is selectively activated in target tissues, such as cancerous cells: this controls the expression of a heterologous enzyme. This molecular chimaera may be manipulated via suitable vectors and incorporated into an infective virion. Upon administration of an infective virion containing the molecular chimaera to a patient, the enzyme is selectively expressed in the target cell. Administration of compounds which are selectively metabolised by the enzyme into metabolites which are either further metabolised to, or are infact cytotoxic or cytostatic agents can then be achieved in situ.

The invention is generally applicable and is demonstrated with respect to hepatocellular carcinoma.

As mentioned above the overwhelming percentage of patients suffering from hepatocellular carcinoma die from the primary tumour. However, approximately 90% of HCC patients have overt metastatic disease at time of death. These metastases exhibit the typical phenotype of the primary tumour and as such these metastases will also selectively express the heterologous enzyme and thus selectively activate administered compounds as herein defined to cytotoxic or cytostatic metabolites.

A number of enzyme pro-drug combinations may be used; providing the enzyme is capable of selectively activating the administered compound either directly or through an intermediate to a cytostatic or cytotoxic metabolite. Equally the choice of compound will depend on the enzyme system used, but must be selectively metabolised by the enzyme either directly or indirectly to a cytotoxic or cytostatic metabolite. The term heterologous enzyme as used herein, refers to an enzyme which displays the appropriate characteristics of selectivity.

The varicella zoster virus (VZV) encodes a specific thymidine kinase protein. The gene has been cloned, sequenced and characterised (J. Gen. Virol. 67: 1759-1816 (1986)). The VZV thymidine kinase will, in contrast to the mammalian enzyme, selectively monophosphorylate specific purine arabinosides and substituted pyrimidine compounds. Moreover, the present inventors have found that certain purine and pyrimidine analogues particularly those of formulae (I) and (II) as hereinafter defined are converted to cytotoxic or cytostatic metabolites in specific mammalian cells which are genetically modified to selectively express VZV thymidine kinase. For example 9-(β-D-arabinofuranosyl)-6-methoxy-9H-purine is converted to 9-β-D-arabinofuranosyl adenine triphosphate [Ara ATP].

Other enzyme pro-drug combinations include the bacterial (for example from Pseudomonas) enzyme carboxypeptidase G2 with the prodrug para-N-bis (2-chloroethyl) aminobenzoyl glutamic acid. Cleavage of the glutamic acid moiety from this compound releases a toxic benzoic acid mustard; alkaline phosphotase from, for example, calf intestine, will convert inactive phosphorylated compounds such as etoposide-phosphate, doxorubicin- phosphate, mitomycin phosphate, to toxic dephosphorylated metabolites. Penicillin-V amidase will convert phenoxyacetamide derivatives of doxorubicin and melphalan to toxic metabolites and the fungal (for example from Fusarium oxysporum) cytosine deaminase will convert 5-fluorocytosine to toxic 5-fluorouracil.

In mammalian cells, certain genes are ubiquitously expressed. Most genes, however, are expressed in a temporal and/or tissue-specific manner, or are activated in response to extracellular inducers, for example certain genes are actively transcribed only at very precise times in ontogeny in specific cell types or in response to some inducing stimulus. This regulation is mediated in part by the interaction between transcriptional regulatory sequences (which are for example promoter and enhancer regulatory DNA sequences), and sequence-specific, DNA-binding transcriptional protein factors.

The present inventors have found that it is possible to alter certain mammalian tissues, eg. liver tissue or transformed liver tissue, to selectively express a heterologous enzyme as herein before defined, eg. VZV thymidine kinase. This is achieved by the construction of molecular chimaeras in an expression cassette.

Expression cassettes themselves are well known in the art of molecular biology. Such an expression cassette will contain all essential DNA sequences required for expression of the heterologous enzyme in a mammalian cell. For example, a preferred expression cassette will contain a molecular chimaera containing the coding sequence for VZV TK, an appropriate polyadenylation signal for a mammalian gene (ie. a polyadenylation signal which will function in a mammalian cell), and suitable enhancers and promoter sequences in the correct orientation.

In mammalian cells, normally two DNA sequences are required for the complete and efficient transcriptional regulation of genes that encode messenger RNAs in mammalian cells: promoters and enhancers. Promoters are located immediately upstream (5') from the start site of transcription. Promoter sequences are required for accurate and efficient initiation of transcription. Different gene-specific promoters reveal a common pattern of organisation. A typical promoter includes an AT-rich region called a TATA box (which is located approximately 30 base pairs 5' to the transcription initiation start site) and one or more upstream promoter elements (UPE). The UPEs are a principle target for the interaction with sequence-specific nuclear transcriptional factors. The activity of promoter sequences are modulated by other sequences called enhancers. The enhancer sequence may be a great distance from the promoter in either an upstream (5') or downstream (3') position. Hence, enhancers operate in an orientation- and position-independent manner. However, based on similar structural organisation and function that may be interchanged the absolute distinction between promoters and enhancers is somewhat arbitrary. Enhancers increase the rate of transcription from the promoter sequence. It is predominantly the interaction between sequence-specific transcriptional factors with the UPE and enhancer sequences that enable mammalian cells to achieve tissue-specific gene expression. The presence of these transcriptional protein factors (tissue-specific, trans-activating factors) bound to the UPE and enhancers (cis-acting, regulatory sequences) enable other components of the transcriptional machinery, including RNA polymerase, to initiate transcription with tissue-specific selectivity and accuracy.

The selection of the transcriptional regulatory sequence, in particular the promoter and enhancer sequence will depend on the targeted tissue. Examples include, the albumin (ALB) and alpha-fetoprotein (AFP) transcriptional regulatory sequence (for example, the promoter and enhancer) specific for normal hepatocytes and transformed hepatocytes respectively; the transcriptional regulatory sequence for carcino-embryonic antigen (CEA) for use in transformed cells of the gastrointestinal tract, lung, breast and other tissues; the transcriptional regulatory sequence for tyrosine hydroxylase, choline acetyl transferase or neuron specific enolase for use in neuroblastomas; the transcriptional regulatory sequence for glial fibro acidic protein for use in gliomas; the transcriptional regulatory sequence for insulin for use in tumours of the pancreas.

Further examples include the transcriptional regulatory sequence specific for gama-glutamyltranspeptidase for use in certain liver tumours and dopa decarboxylase for use in certain tumours of the lung.

In addition the transcriptional regulatory sequences from certain oncognes may be used as these are expressed predominantly in certain tumour types. Good examples of these include the HER-2/neu oncogene regulatory sequence which is expressed in breast tumours and the regulatory sequence specific for the N-myc oncogene for neuroblastomas.

The ALB and AFP genes exhibit extensive homology with regard to nucleic acid sequence, gene structure, amino acid sequence and protein secondary folding (for review see Ingram et al PNAS 78 4694-4698 (1981). These genes are independently but reciprocally expressed in ontogeny. In normal development ALB transcription is initiated shortly before birth and continues throughout adulthood. Transcriptional expression of ALB in the adult is confined to the liver. AFP is normally expressed in fetal liver, the visceral endoderm of the yolk sac and the fetal gastrointestinal tract, but declines to undetectable levels shortly after birth and is not significantly expressed in nonpathogenic or non-regenerating adult liver or in other normal adult tissues. However, AFP transcription in adult liver often increases dramatically in HCC. In addition, AFP transcription may also be elevated in non-seminomatous and mixed carcinoma of the testis; in endodermal sinus tumours, in certain tertocarcinomas, and in certain gastrointestinal tumours. Liver-specific expression of AFP and ALB is the result of interactions of the regulatory sequences of their genes with trans-activating transcriptional factors found in nuclear extracts from liver. The AFP and ALB transcriptional regulatory sequences are preferred for generating hepatoma-specific or general liver-specific, expression respectively, of molecularly combined genes since the AFP and ALB genes are regulated at the transcriptional level and their mRNAs are among the most abundant polymerase II transcripts in the liver.

Several mammalian ALB and AFP promoter and enhancer sequences have been identified (for review see Genes and Develop. 1: 268-276 (1987); Science 235: 53-58 (1987); The Journal of Biol.Chemistry 262: 4812-4818 (1987). These sequences enable the selective and specific expression of genes in liver hepatocytes (normal and transformed) and hepatomas, respectively.

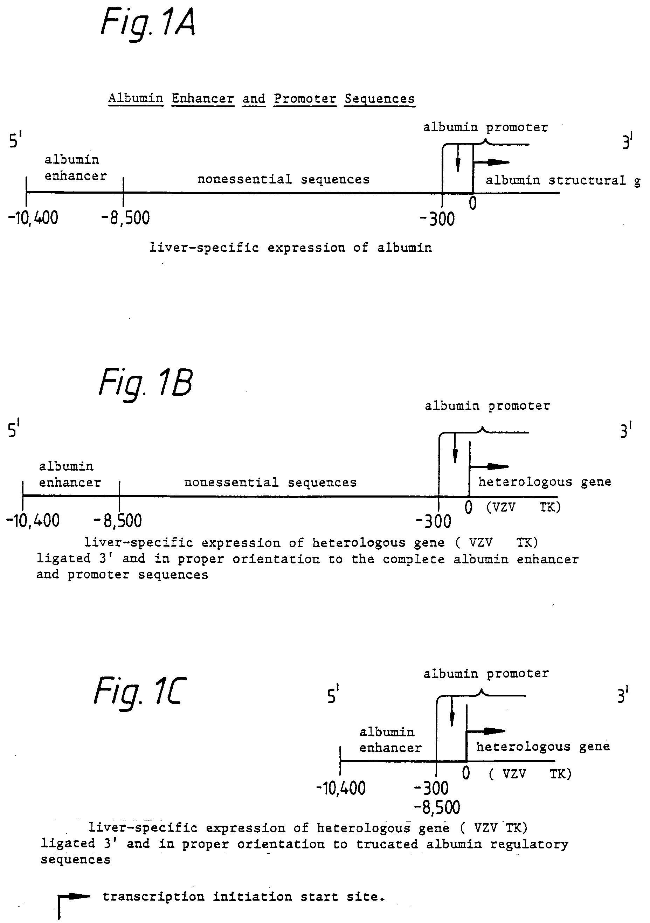

For example as shown in figure 1 a mammalian ALB promoter is contained within 300 bp fragment 5' to the transcription initiation start site of the albumin gene. The sequence contained between 300 bp 5' and 8,500 bp 5' to the transcription initiation start site of the murine albumin gene is dispensable for liver-specific expression. However, a liver-specific enhancer sequence is contained in a fragment located from 8,500 bp 5' to 10,400 bp 5' to the transcription initiation start site (Fig 1A). If the ALB promoter and enhancer elements are present, liver-specific expression of a heterologous structural gene positioned in a proper 3' orientation can be achieved(Fig 1B). Liver-specific expression of a 3' heterologous structural gene positioned in the proper orientation to the ALB promoter and enhancer sequences is also maintained when the nonessential intervening sequences located between 300 bp 5' and 8,500 bp 5' to the transcription initiation start site are eliminated (Fig 1C). The truncation of nonessential sequences is accomplished by standard molecular biological methodology well known in the art and results in a molecular chimaera that can be used to direct liver-specific expression of VZV TK.

Similar to the regulatory structure of the ALB gene, the regulatory elements of the AFP genes promote tissue-specific expression of AFP in certain liver pathologies, such as HCC (Mol.Cel.Biol. 6: 477-487 (1986); Science 235: 53-58 (1987)). The regulatory elements of a mammalian AFP gene consist of a specific 5' promoter-proximal region (located in some mammalian species between 85 and 52 bp 5' to the gene). This sequence is essential for transcription in hepatomas. In addition, there are upstream (5') regulatory elements well defined for the murine AFP gene which behave as classical enhancers (Mol.Cel.Biol. 6: 477-487 (1986); Science 235: 53-58 (1987)). These upstream regulatory elements are designated elements I, II, and III and are located between 1,000 to 7,600 bp 5' to the transcription initiation site for the AFP murine gene. These three enhancer domains are not functionally equivalent at generating tissue-specific expression of AFP. Elements I and II have the greatest capacity to direct liver-specific expression of AFP. It is important to note that the regulatory sequences of the alpha-fetoprotein gene advantageously contain the sequences not only for tissue-specific transcriptional activation but also for repression of expression in tissues which should not express AFP. In a similar fashion the regulatory regions of the human alpha-fetoprotein gene have been characterised (J.B.C. 262 4812 (1987)). A structural gene placed in the correct orientation 3' to the AFP regulatory sequences will enable that structural gene to be selectively expressed in fetal liver, hepatomas, non seminamatous carcinomas of the testis, certain teratocarcinomas, certain gastrointestinal tumours and other normal and pathological tissues which specifically express AFP.

The present invention provides a molecular chimaera comprising a DNA sequence containing the coding sequence of the gene coding for a heterologous enzyme under the control of a transcriptional regulatory sequence in an expression cassette; the promoter capable of functioning selectively in a target cancer cell; for example one which is capable of transforming a cancer cell to selectively express VZV thymidine kinase.

The present invention further provides in a preferred embodiment, a molecular chimaera comprising a transcriptional regulatory sequence, in particular a promoter which is selectively and activated in mammalian target tissues and operatively linked to the coding sequence for the gene encoding varicella zoster virus thymidine kinase (VZV TK).

In particular, the present invention provides a molecular chimaera comprising a DNA sequence of the coding sequence of the gene coding for the enzyme, which is preferably VZV TK, including an appropriate polyadenylation sequence, linked in a 3' position and in the proper orientation to a mammalian target tissue specific transcriptional regulatory sequence. Most preferably the expression cassette also contains an enhancer sequence.

The promoter and enhancer sequences, preferably, are selected from the transcriptional regulatory sequence for one of albumin (ALB), alpha-fetoprotein (AFP), carcino-embryonic antigen (CEA), tryrosine hydroxylase, choline acetyl transferase, neuron-specific enolase, glial fibro acid protein, insulin or gama-glutamyltranspeptidase, dopadecarboxylase, HER2/neu or N-myc oncogene or other suitable genes. Most preferably the regulatory sequence for ALB or AFB are used to direct liver specific or hepatoma specific expression respectively.

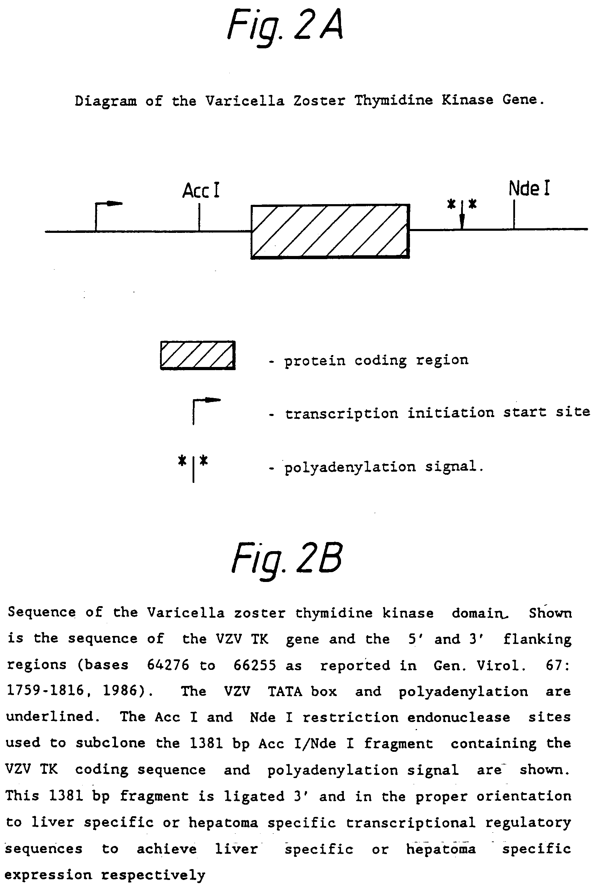

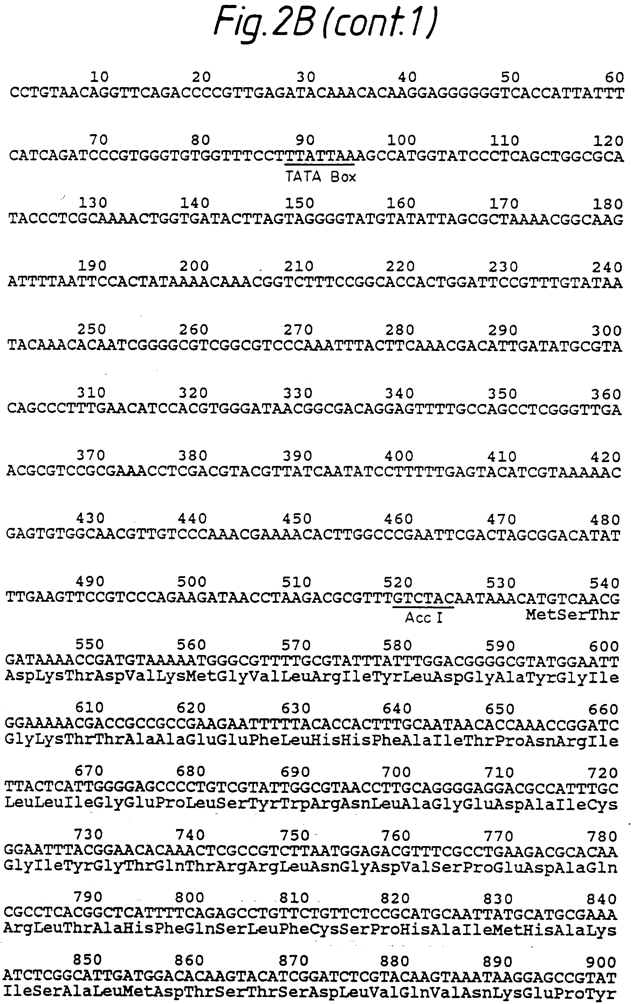

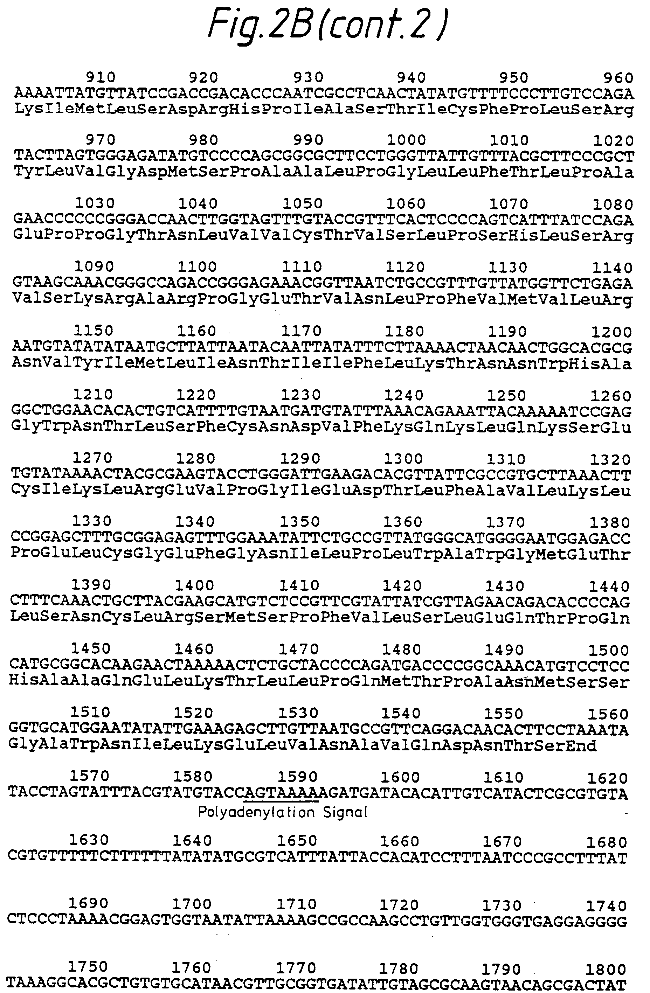

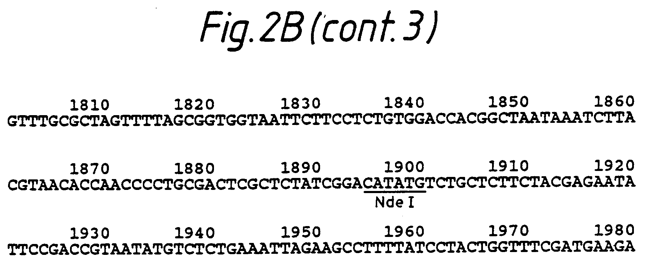

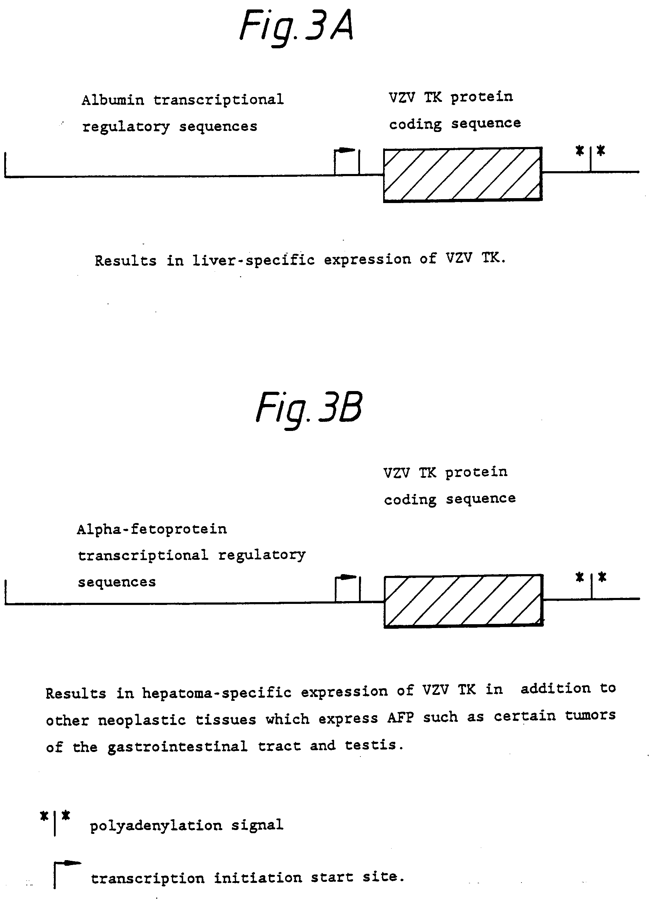

The molecular chimaera of the present invention may be made utilising standard recombinant DNA techniques. Thus the coding sequence and polyadenylation signal of, for example, the VZV thymidine kinase (TK) gene (see Figs. 2A and 2B) is placed in the proper 3' orientation to the essential, ALB or AFP transcriptional regulatory elements. These molecular chimaeras enable the selective expression of VZV TK in cells which normally express ALB or AFP, respectively (Figure 3A and 3B). Expression of the VZV TK gene in mammalian liver, hepatomas, certain tumours of the gastrointestinal tract, nonseminamatous carcinomas of the testis, certain teratocarcinomas and other tumours will enable relatively nontoxic arabinosides and pyrimidines as herein defined to be selectively metabolised to cytotoxic and/or cytostatic metabolites.

Accordingly, in a second aspect, there is provided a method of constructing a molecular chimaera comprising linking a DNA sequence encoding a heterologous enzyme gene, eg. VZV TK to a tissue specific promoter.

In particular the present invention provides a method of constructing a molecular chimaera as herein defined, the method comprising ligating a DNA sequence encoding the coding sequence and polyadenylation signal of the gene for a heterologous enzyme, eg. VZV thymidine kinase to a mammalian tissue specific transcriptional regulatory sequence (eg. promoter sequence and enhancer sequence).

The VZV thymidine kinase coding sequence and 3' polyadenylation signal reside in an approximately 1,381 bp AccI/Nde I restriction endonuclease fragment (see Figure 2).

Preferably it is the 1381bp AccI/Nde I fragment containing the VZV TK coding sequence and polyadenylation signal that is ligated to the mammalian tissue specific promoter and enhancer sequences, although it will be appreciated that other DNA fragments containing the VZV TK gene could be used. Moreover, the VZV TK polyadenylation signal could be replaced with another suitable polyadenylation signal such as from the SV40 virus or other mammalian genes.

Preferably the promoter and enhancer sequences are selected from the transcriptional regulatory sequences for one of albumin (ALB), alpha-fetoprotein (AFP), carcino-embryonic antigen (CEA), tryrosine hydroxylase, choline acetyl transferase, neuron-specific enolase, glial fibro acidic protein, insulin, gama glutamyltranspeptidase, dopa decarboxylase, HER2/neu or N-myc oncogenes or other suitable genes.

These molecular chimaeras can be delivered to the target cell by a delivery system. For administration to a patient, it is necessary to provide an efficient in vivo delivery system which stably integrates the molecular chimaera into the cells. Known methods utilise techniques of calcium phosphate transfection, electroporation, microinjection, liposomal transfer, ballistic barrage or retroviral infection. For a review of this subject see Biotechnique Vol.6. No.7. 1988.

The technique of retroviral infection of cells to integrate artificial genes employs retroviral shuttle vectors which are known in the art, (see for example Mol. and Cell Biol Aug 86 p2895). Essentially, retorviral shuttle vectors are generated using the DNA form of the retrovirus contained in a plasmid. These plasmids also contain sequences necessary for selection and growth in bacteria. Retroviral shuttle vectors are constructed using standard molecular biology techniques well known in the art. Retroviral shuttle vectors have the parental endogenous retroviral genes (eg. gag, pol and env) removed and the DNA sequence of interest inserted, such as the molecular chimaeras which have been described. They however, contain appropriate retroviral regulatory sequences for viral encapsidation, proviral insertion into the target genome, message splicing, termination and polyadenylation. Retroviral shuttle vectors have been derived from the Moloney murine leukemia virus (Mo-MLV) but it will be appreciated that other retroviruses can be used such as the closely related Moloney murine sarcoma virus. Certain DNA viruses may also prove to be useful as a delivery system. The bovine papilloma virus [BPV] replicates extrachromosomally so that delivery systems based on BPV have the advantage that the delivered gene is maintained in a nonintegrated manner.

Thus according to a third aspect of the present invention there is provided a retroviral shuttle vectors containing the molecular chimaeras as hereinbefore defined.

The advantages of a retroviral-mediated gene transfer system are the high efficiency of the gene delivery to the targeted tissue, sequence specific integration regarding the viral genome (at the 5' and 3' long terminal repeat (LTR) sequences) and little rearrangements of delivered DNA compared to other DNA delivery systems.

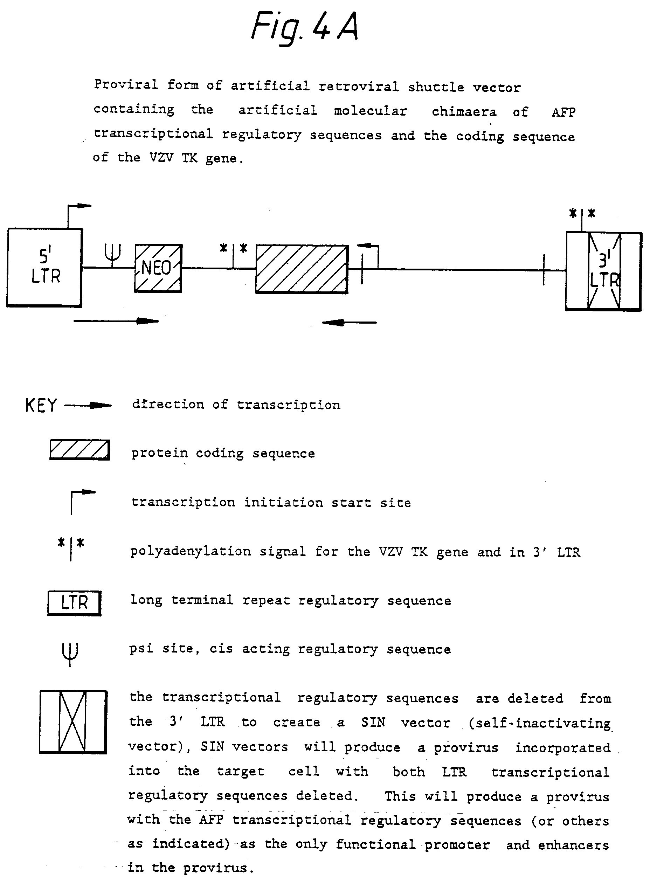

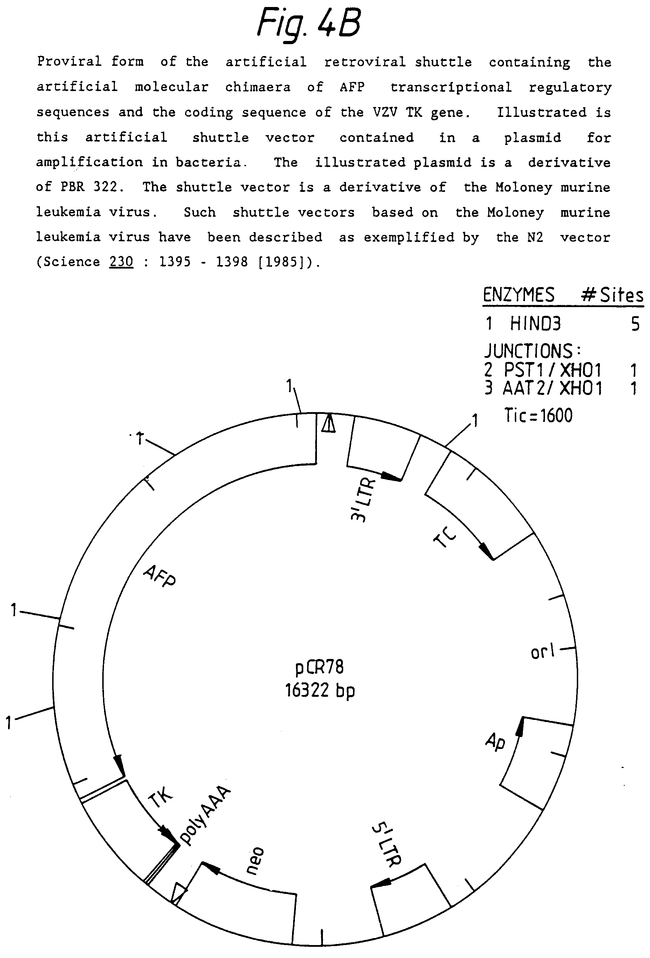

Accordingly in a preferred embodiment of the present invention there is provided a retroviral shuttle vector comprising a DNA sequence comprising a 5' viral LTR sequence, a cis acting psi encapsidation sequence, a molecular chimaera as hereinbefore defined and a 3' viral LTR sequence (figure 4A and figure 4B).

In a preferred embodiment, and to help eliminate non-tissue-specific expression of the molecular chimaera, the molecular chimaera is placed in opposite transcriptional orientation to the 5' retroviral LTR (figure 4A and figure 4B). In addition, a dominant selectable marker gene may also be included which is transcriptionally driven from the 5' LTR sequence. Such a dominant selectable marker gene may be the bacterial neomycin-resistance gene NEO (Aminoglycoside 3' phosphotransferase type II), which confers on eukaroytic cells resistance to the neomycin analogue G418 sulphate (geneticin) (trade mark), (Figure 4A and 4B). The NEO gene aids in the selection of packaging cells which contain these sequences (see below).

The retroviral vector used in the examples is based on the Moloney murine leukemia virus but it will be appreciated that other vectors may be used. Such vectors containing a NEO gene as a selectable marker have been described, for example, the N2 vector (Science 230 : 1395-1398 (1985)).

A theoretical problem associated with retroviral shuttle vectors is the potential of retroviral long terminal repeat (LTR) regulatory sequences transcriptionally activating a cellular oncogene at the site of integration in the host genome. This problem may be diminished by creating SIN vectors (figure 4A). SIN vectors are self-inactivating vectors which contain a deletion comprising the promoter and enhancer regions in the retroviral LTR. The LTR sequences of SIN vectors do not transcriptionally activate 5' or 3' genomic sequences. The transcriptional inactivation of the viral LTR sequences diminishes insertional activation of adjacent target cell DNA sequences and also aids in the selected expression of the delivered molecular chimaera. SIN vectors are created by removal of approximately 299 bp in the 3' viral LTR sequence (Biotechniques 4 504-512 (1986)).

Thus preferably the retroviral shuttle vector of the present invention are SIN vectors.

Since the parental retroviral gag, pol and env genes have been removed from these shuttle vectors, a helper virus system may be utilised to provide the gag, pol and env retroviral gene products in trans to package or encapsidate the retroviral vector into an infective virion. This is accomplished by utilising specialised "packaging" cell lines, which are capable of generating infectious, synthetic virus yet are deficient in the ability to produce any detectable wild-type virus. In this way the artificial synthetic virus contains a chimaera of the present invention packaged into synthetic artificial infectious virions free of wild-type helper virus. This is based on the fact that the helper virus that is stably integrated into the packaging cell contains the viral structural genes, but is lacking the psi site, a cis acting regulatory sequence which must be contained in the viral genomic RNA molecule for it to be encapsidated into an infectious viral particle.

Accordingly in a fourth aspect of the present invention there is provided an infective virion comprising a retroviral shuttle vector as hereinbefore described, said vector being encapsidated within viral proteins to create an artificial infective, replication-defective retrovirus.

Preferably the retroviral shuttle vector comprises a shuttle vector comprising a molecular chimaera having the transcriptional regulatory sequence of AFP or ALB. In particular, the shuttle vector contains a AFP/VZV TK chimaera or ALB/VZV TK chimaera.

In addition to removal of the psi site, additional alterations can be made to the helper virus LTR regulatory sequences to ensure that the helper virus is not packaged in virions and is blocked at the level of reverse transcription and viral integration.

Alternatively, helper virus structural genes (i.e. gag, pol and env) may be individually and independently transferred into the packaging cell line. Since these viral structural genes are separated within the packaging cell's genome, there is little chance of covert recombinations generating wild-type virus.

In a fifth aspect of the present invention there is provided a method for producing infective virions of the present invention by delivering the artificial retroviral shuttle vector comprising a molecular chimaera of the invention, as hereinbefore described into a packaging cell line.

The packaging cell line may have stably integrated within it a helper virus lacking a psi site and other regulatory sequence as hereinbefore described, or alternatively the packaging cell line may be engineered so as to contain helper virus structural genes within its genome.

The present invention further provides an infective virion as hereinbefore described for use in therapy, particularly for use in the treatment of cancer and more particularly for use in the treatment of HCC, nonseminomatous carcinoma of the testis, certain teratocarcinomas and certain gastrointestinal tumours.

Selective expression of the heterologous enzyme, in particular VZV thymidine kinase gene is accomplished by utilising tissue-specific, transcriptional regulatory (eg. enhancer and promoter) sequences. Selectivity may be additionally improved by selective infection of liver cells. The retroviral env gene present in the packaging cell line defines the specificity for host infection. The env gene used in constructing the packaging cell line is modified to generate artificial, infective virions that selectively infect hepatocytes. As an example a retroviral env gene introduced into the packaging cell may be modified in such a way that the artificial, infective virion's envelope glycoprotein selectively infect hepatocytes via the specific receptor mediated binding utilised by the hepatitis B virus (HBV).

HBV primarily infects hepatocytes via specific receptor mediated binding. The HBV proteins encoded by the pre-S1 and pre-S2 sequences play a major role in the attachment of HBV to hepatocytes (Hepadna Viruses edited Robinson et al 189-203, 205-221, 1987). The env gene of the packaging cell is modified to include the hepatocyte binding site of the large S HBV envelope protein. Such modifications of the env gene introduced into the packaging cell may be performed by standard molecular biology techniques well known in the art and will facilitate viral uptake in the target tissue.

The infective virion according to the invention may be formulated by techniques well known in the art and may be presented as a formulation with a pharmaceutically acceptable carrier therefor. Pharmaceutical acceptable carriers, in this instance, may comprise a liquid medium suitable for use as vehicles to introduce the infective virion into the patient. An example of such a carrier is saline. The infective virion may be a solution or suspension in such a vehicle. Stabilisers and antioxidants and/or other excipients may also be present in such pharmaceutical formulations which may be administered to a mammal by any conventional method eg oral or parenteral routes. In particular, the infective virion may be administered by intra-venous or intra-arterial infusion. In the case of treating HCC intra-hepatic arterial infusion may be advantageous.

Accordingly the invention provides a pharmaceutical formulation comprising an infective virion in admixture with a pharmaceutically acceptable carrier.

Additionally, the present invention provides methods of making pharmaceutical formulations as herein described comprising mixing an artificial infective virion, containing a molecular chimaera, with a pharmaceutically acceptable carrier.

Whilst any suitable compound which can be selectively converted by the enzyme may be utilised, the present invention further provides the use of compounds of formula I or II in the manufacture of a medicament for use in treating cancers capable of expressing VZV thymidine kinase. In particular for use in treating hepatocellular carcinoma (HCC).

6-Substituted purine arabinosides of formula (I) it salts and physiologically functional equivalents thereof as shown hereinbelow: 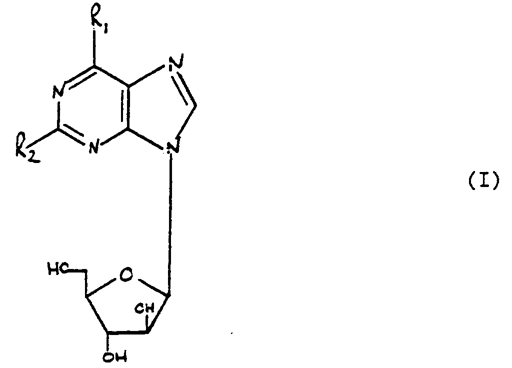
wherein
R₁ is halo, C₁₋₅ alkoxy, halogen-substituted C₁₋₅ alkoxy; an amino group which is mono -or di- substituted by C₁₋₅ alkyl, C₁₋₅ alkyl substituted by one or more fluorine atoms, C₃₋₆ cycloalkyl, or a nitrogen containing heterocycle containing C₄₋₇ carbon atoms and optionally a double bond; and R₂ is hydrogen, halo or amino are known as anti VZV and cytomegalovirus agents and their use and synthesis are described in European patent application published under No. 0294114.

Compounds of formula II are as shown hereinbelow 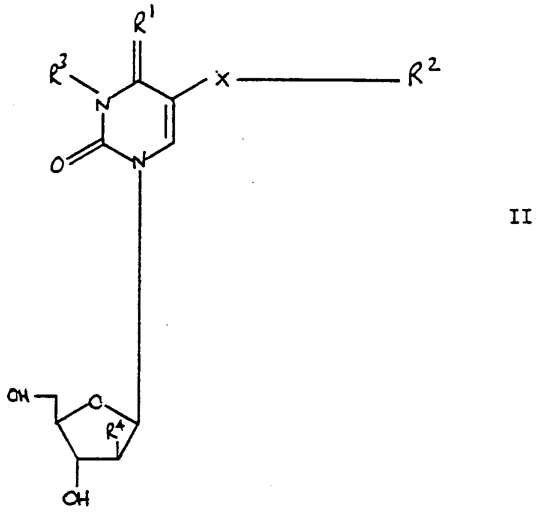
wherein X represents a vinylene or ethynylene group; R¹ represents an oxo or imino group; R² represents a hydrogen atom, C₁₋₂ alkyl, C₃₋₄ branched or cycloalkyl group e.g. isopropyl or cyclopropyl; R³ represents a hydrogen atom or an acyl e.g. C₁₋₄ alkanoyl or benzoyl group optionally substituted for example by one or more halogen, alkyl, hydroxy or alkoxy substituents; and R⁴ represents a hydrogen atom or a hydroxy group; providing that (a) when R², R³ and R⁴ each represents a hydrogen atom, R¹ does not represent an oxo group.

It will be appreciated that when R³ is not an acyl group, the compound of formula (I) or (II) may exist in its tautomeric form. Particularly preferred compounds of formula I and II are 9-β-Arabinofuranosyl-6-methoxy-9H-purine and 1-(β-D-arabinofuranosyl)-5-propynluracil.

These compounds and methods of their synthesis have been disclosed in European Patent application published under No. 0272065 as having anti VZV activity.

The above-mentioned pyrimidine nucleosides and purine arabinosides also include the pharmaceutically acceptable derivatives of such compounds, ie. any pharmaceutically acceptable salt, ester, or salt of such ester, or any other compound which, upon administration to a human subject, is capable of providing (directly or indirectly) the active metabolite or residue thereof. Preferably the compound is orally active.

The amounts and precise regime in treating a mammal, will of course be the responsibility of the attendant physician, and will depend on a number of factors including the type and severity of the condition to be treated. However, for HCC, an intrahepatic arterial infusion of the artificial infective virion at a titre of between 2 x 10⁵ and 2 x 10⁷ colony forming units per ml (CFU/ml) infective virions is likely to be suitable for a typical tumour. Total amount of virions infused will be dependent on tumour size and would probably be given in divided doses.

Drug treatment - Subsequent to infection with the infective virion, compounds according to the invention are administered which specifically require VZV TK activity for the critical first phosphorylation step in anabolism to cytotoxic or cytostatic metabolites.

The dose of drug will advantageously be in the range 0.1 to 250 mg per kilogram body weight of recipient per day, preferably 0.1 to 100mg per kilogram bodyweight.
The following examples serve to illustrate the present invention but should not be construed as a limitation thereof:

### Example 1

### Construction of transcriptional regulatory sequence of albumin / VZV thymidine kinase molecular chimaera

An approximately 1381 bp Acc I / Nde 1 DNA fragment (all restriction enzymes obtained from either Bethesda Research Laboratories, Gaithersburg MD, USA; New England Biolabs, Mass, USA; or Promega, Madison, Wi, USA; all enzymatic reactions performed as specified by the supplier) containing the coding sequence and polyadenylation signal of the VZV TK gene was purified by electroelution using an elutrap electrophoresis chamber (by Schleicher and Schuell, Keene NH, USA) from a restriction endonuclease digestion of an approximately 4896 bp plasmid, designated 22TK, containing an approximately 2200 bp EcoRI/BamHI fragment of the VZV TK genome (Virology 149: 1-9, 1986; supplied by J.Ostrove, NIH, Bethesda, Md.). The purified DNA fragment contains the entire VZV TK coding sequence and polyadenylation signal, but does not include any VZV TK promotional elements (see Fig. 2A and 2B).

The 5' overhanding ends of the purified 1381 bp AccI/Nde I VZV TK fragment were made blunt by treatment with the Klenow fragment of E.coli DNA polymerase I (Bethesda Research Laboratories, Gaithersburg, MD, USA) and deoxynucleotide triphosphate (dNTPs).

An approximately 5249 bp plasmid, designated 2335A-1, containing approximately 2300 bp of a ALB E/P sequence was obtained from Richard Palmiter (University of Washington, Seatle, Washington, USA). This construct contains sequences necessary for liver-specific expression but lacks nonessential intervening sequences. A unique BamH I restriction endonuclease recognition site is present at +23 relative to the start of transcription. 2335A-1 was digested with the restriction endonuclease BamH I and the 5' overhanging ends were made blunt by treatment with Klenow and dNTPs as described above.

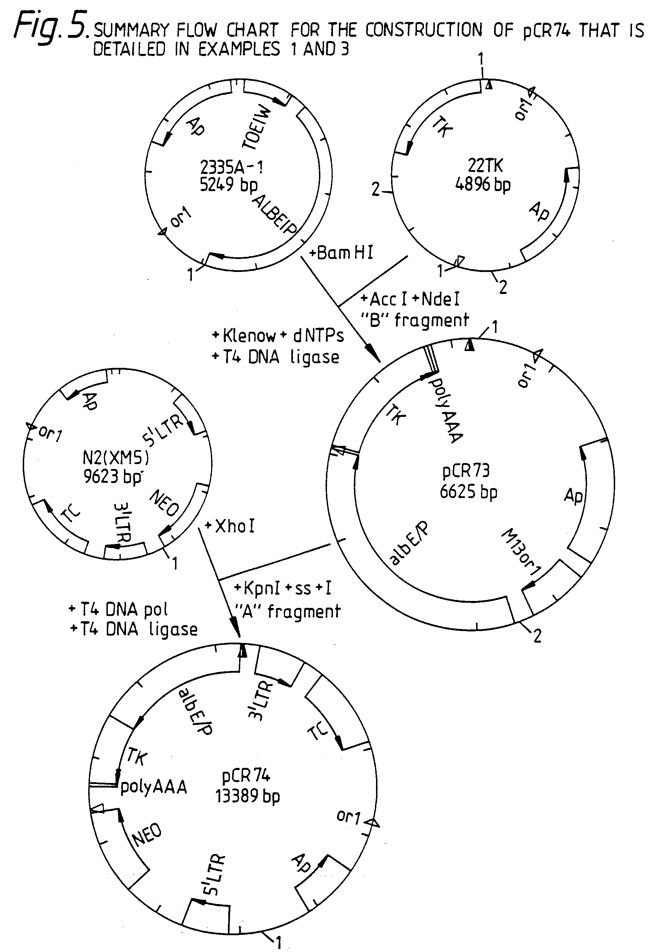

The ALB E/P VZV TK chimaera was constructed by ligating the blunt ended AccI-NdeI fragment containing the VZV TK coding and polyadenylation sequences into the blunt ended BamH I site of 2335A-1 creating pCR73 using T4 DNA ligase (Bethesda Research Laboratories, Gaithersburg, MD) (Figure 5). Similar to all ligations described, the orientation of the ligated fragments was determined by restriction endonuclease digestions by methods well known in the art. Similar to all plasmids described in all examples, pCR73 contains essential sequences required for replication in bacteria and suitable for amplification by methods well known in the art. The ALB E/P VZV TK chimaera was purified by electroelution from pCR73 as an approximately 3880 bp SstI/KpnI restriction endonuclease fragment (Figure 5). The 3' overhanging ends were made blunt by treatment with T4 DNA polymerase and dNTPs. This SstI/KpnI blunt ended restriction fragment was subsequently introduced into a Moloney murine leukemia virus retroviral shuttle vector system (see below, example 3).

pCR73 was deposited at the ATTC on 18th August 1989 under Accession No. ATTC68077.

### Example 2

### Construction of transcriptional regulatory sequence of alpha-fetoprotein/VZV thymidine kinase

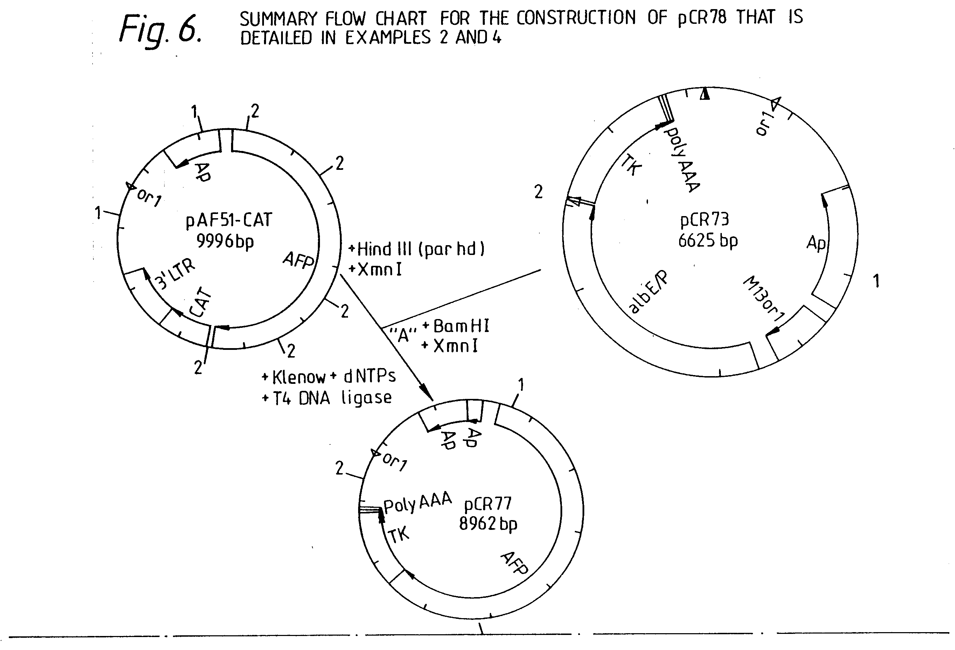

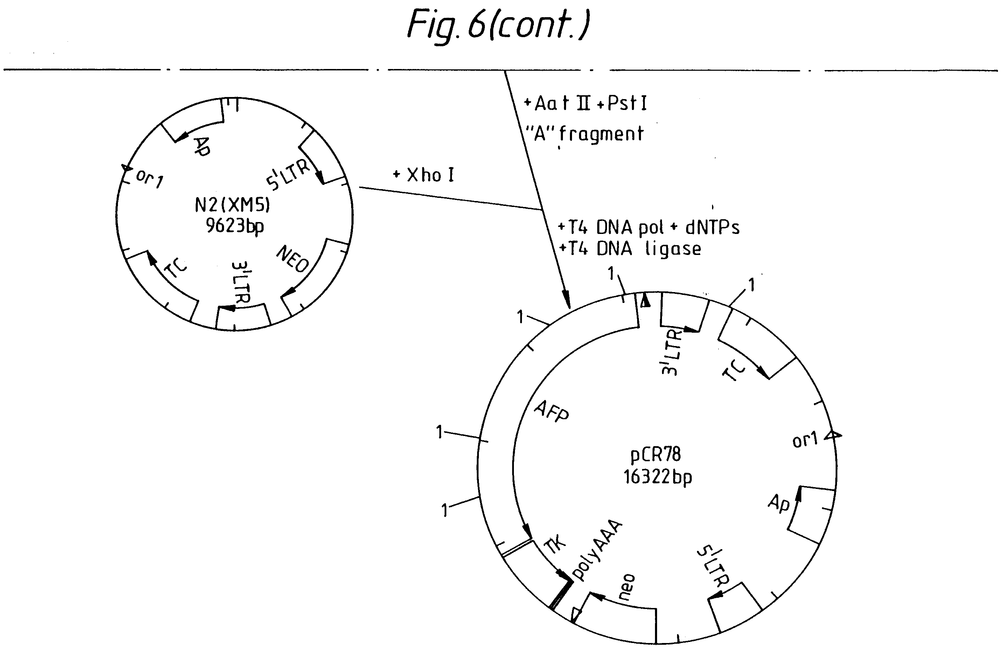

The VZV thymidine kinase coding sequence and polyadenylation site was isolated as an approximately 3,300 bp BamHI-XmnI restriction endonuclease fragment of pCR73 (figure 6). The 5' overhanging end of the BamHI restriction endonuclease site was made blunt by treatment with Klenow and dNTPs. This DNA fragment contains the complete coding sequence and the polyadenylation site of the VZV TK gene but does not contain any enhancer or promoter sequences. An approximately 9996 bp plasmid, pAF5.1-CAT, containing an approximately 5,100 bp of human AFP 5' flanking DNA was obtained from T.Tamaoki, Univ. of Calgary, Canada. A DNA fragment spanning from approximately -5.1 kb to +29 of the human AFP gene was isolated from pAF5.1-CAT by digestion with XmnI and partial digestion with HindIII. This XmnI/HindII fragment was ligated to the BamHI/XmnI VZV TK fragment using T4 DNA ligase to form pCR 77 (figure 6). The AFP E/P VZV TK chimera was purified by electroelution from PCR 77 as an approximately 6699 bp Aat II /PstI restriction endonuclease fragment. This fragment was then treated with T4 DNA polymerase and dNTPs as example 1 to produce a blunt end restriction fragment.

pCR77 was deposited at the ATCC on 18th August 1989, under Accession No. ATCC68079.

### Example 3

### Construction of a retroviral shuttle vector construct containing the molecular chimaera of example 1.

The retroviral shuttle vector, pCR74, containing the ALB E/P VZV TK chimaera was constructed by ligating the purified SstI/KpnI blunt ended fragment of pCR73 into a Moloney murine leukemia retroviral vector designated N2(XM5) (Science 230: 1395-1398, 1985) obtained from S.Karrlson, NIH, Bethesda, MD, USA. N2(XM5) was digested with the restriction endonuclease XhoI and the 5' overhanging ends were made blunt by treatment with both Klenow and dNTPs prior to the ligation to the pCR73 SstI/KpnI fragment using T4 DNA ligase (Figure 5).

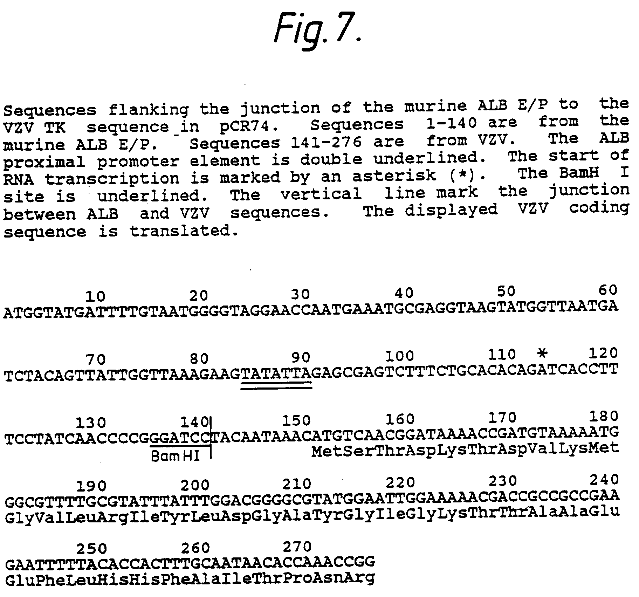

The retroviral shuttle vector pCR74 containing the ALB E/P VZV TK chimaera has been characterised by restriction endonuclease mapping and DNA sequencing to confirm the primary sequence. The sequence flanking the junction of the ALB E/P to the VZV TK sequences is shown in Figure 7.

pCR74 was deposited at the ATCC on 18th August 1989 under Accession No. ATCC68078.

### Example 4

### Construction of a retroviral shuttle vector construct containing the molecular chimaera of example 2.

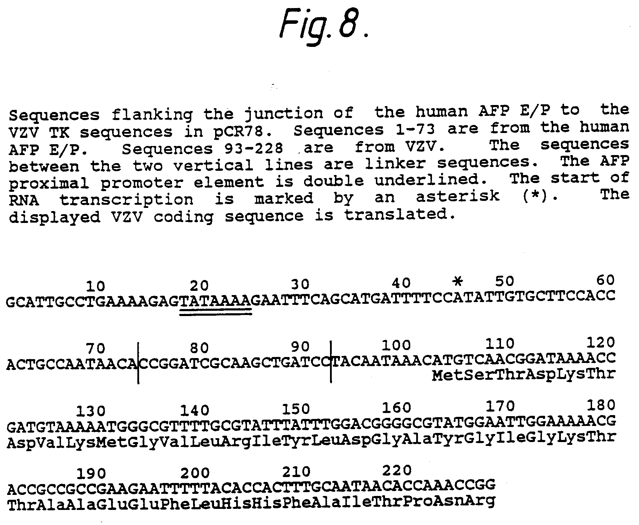

The retroviral shuttle vector pCR78 (figure 6) was constructed by ligating a purified AatII/PstI fragment of pCR77 containing the AFP E/P VZV TK chimaera into N2(XM5) which was digested with XhoI and made blunted ended with T4 DNA polymerase and dNTPs as described in Example 3. The retroviral shuttle vector pCR78 containing the AFP E/P VZV TK chimaera has been characterised by restriction endonuclease mapping and DNA sequencing to confirm the primary sequence. The sequence flanking the junction of AFP E/P to the VZV TK sequence is shown in figure 8.

pCR78 was despoited at the ATCC on 18th August 1989 under Accession No. ATCC68080.

### Example 5

### Virus Production

The packaging cell line called PA317 obtained from ATCC, (ATCC CRL 9078), which has been previously described, has three alterations contained within the 5' LTR, psi regulatory sequence and 3' LTR (Mol. and Cell Biol 6 No.8 2895-2902 [1986]). The artificial retroviral constructs described in example 3 and 4 are placed into the packaging cell line by electroporation or infection. For electroporation, 20ug of linearized plasmid DNA is electroporated into 2 million PA317 cells in phosphate buffered sucrose using 280 volts, 25 microfarads of capacitance in a total volume of 0.8mls. One can obtain at least 150 G418 resistant colonies / 20ug plasmid DNA/2 million PA317 cells. For infection, 20ug of linearized plasmid DNA is electroporated into 2 million ecotropic packaging cells, such as Psi 2 cells. Two days later, the culture supernatant is used to infect the amphotropic packaging cell line PA317 and 418 resistant colonies isolated. For both electroporation and infection techniques, G418 resistant colonies are single cell cloned by the limiting dilution method and analysed by Southern blots and titered in NIH 3T3 cells (ATCC) to identify the highest producer of full length virus. For PA317 cells containing pCR74, 17 single cell clones were isolated and DNA was extracted from 10 of these clones. Extensive Southern blot analysis using different restriction endonuclease enzymes and NEO and VZV TK sequences as radioactive hybridization probes was performed on these 10 DNA samples. Out of the 10 clones analysed, two showed no evidence of truncation and are considered full length. For PA317 cells containing pCR78, 29 single cell clones were isolated and DNA was obtained from 25 clones. Extensive Southern blot analysis using different restriction endonuclease enzymes and AFP, NEO and VZV TK sequences as radioactive hybridisation probes was performed on these 25 samples. Out of the 25 clones analysed, 5 showed no evidence of truncation and are considered full length. Each packaging cell line containing a full length viral sequence was titered in NIH 3T3 cells which were thymidine kinase minus/minus using standard techniques.

### Example 6

### Infection of a human hepatoma cell line (positive control) with full length infective virions of Example 5 containing ALB/VZV TK or AFP/VZV TK with subsequent measurements of VZV TK activity, ara ATP production and drug sensitivity.

The replication-defective, full length, artificial retroviruses containing the ALB/VZV TK chimaera or AFP/VZV TK chimaera were used to infect a human hepatoma cell line called Hep G2 (ATCC HB 8065). Following infection and selection on 1mg geneticin/ml (Trade mark), the cells were assayed for VZV TK activity. In addition, the cells were incubated in the presence of (³H) labeled 9-(B-D-arabinofuranosyl)-6- methoxy-9H purine (designated as ara-M in the following tables and figures) with subsequent measurement of ara ATP. Finally, cells were cultured in the presence of the above mentioned compound and the IC₅₀ (growth inhibition) was determined. Cells infected with no virus or N2 virus act as control samples for these experiments. N2 virus contain no VZV TK genetic material.

Table 1 demonstrates that the human hepatoma cells infected with either pCR74 or pCR78 containing viruses have 904 X or 52 X more VZV TK activity, respectively, compared to control cells.

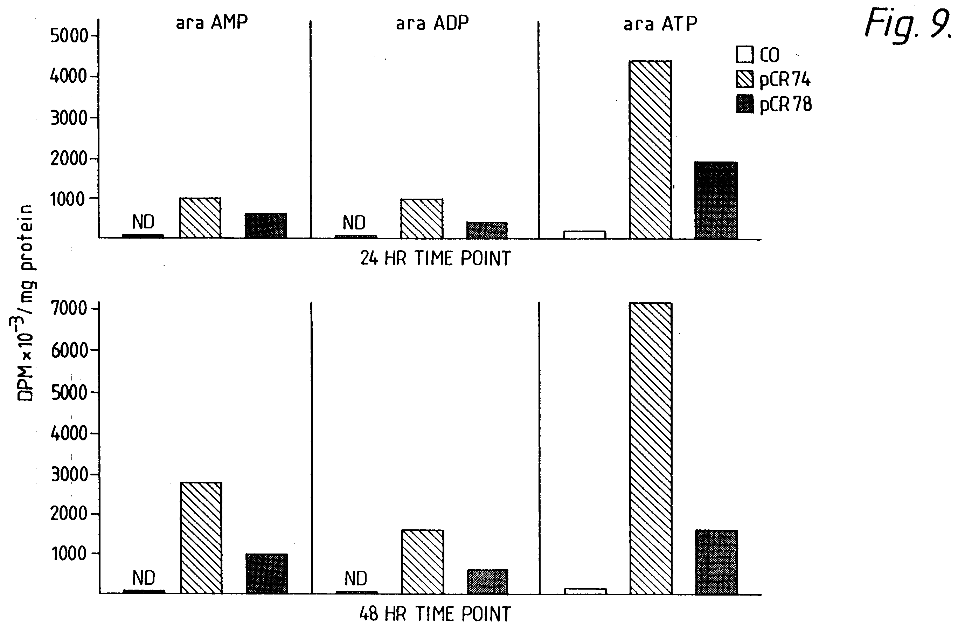

9-(B-D-arabinofuranosyl)-6-methoxy-9H purine (designated as ara-m) can be selectively monophosphorylated by VZV TK with subsequent anabolism to cytotoxic ara ATP. Figure 9 demonstrates that Hep G2 cells which were infected with either pCR74 or pCR78 containing viruses and incubated in the presence of (3H) labelled 9-B-D arabinofuranosyl)-6-methoxy-9H purine had significant amounts of cytotoxic (³H) araATP formation.

Hep G2 cells infected with the replication-defective, full length, artificial retroviruses containing the ALB/VZV TK chimaera (pCR74) or AFP/VZV TK (pCR78) chimaera were incubated in the presence of varying amounts of 9-(B-D-arabinofuranosyl)-6-methoxy-9H purine for 5 days and growth inhibition determined as measured by cell number and DNA content.

Table 2 demonstrates that the IC₅₀ (50% growth inhibition) of 9-(B-D-arabinofuranosyl)-6-methoxy-9H purine is greater than 2000µM in control and N2 infected Hep G2 cells. Hep G2 cells infected with the replication-defective, full length, artificial retroviruses containing the ALB/VZV TK chimaer (pCR74) or AFP/VZV TK (pCR78) chimaeras, the IC₅₀ were 5µM and 175µM, respectively. Single cell cloning of Hep G2 cells containing the AFP/VZV TK (pCR78) chimaera indicated that the IC₅₀ levels of 9-(B-D-arabinofuranosyl)-6-methoxy-9H purine can be further decreased to approximately 40µM.

### Example 7

### Selectivity of Expression of VZV TK.

Five human, non-hepatoma cell lines were infected with replication-defective, full length, artifical retroviruses containing the ALB/VZV TK chimaera (pCR74). These cell lines were WiDR (ATCC CCL218), IMR90 (ATCC CCL186), MCF7 (ATCC HTB22), Detroit 555 (ATCC CCL110) and SW480 (ATCC CCL228). Subsequent to infection and selection on geneticin (Trade mark), these cells were assayed for VZV TK activity and growth inhibition in the presence of 9-(B-D-arabinofuranosyl)-6-methoxy-9H purine, as described above. There was no increase in VZV TK activity or drug sensitivity to 9-(B-D-arabinofuranosyl)-6-methoxy-9H purine in these five non-hepatoma cell lines infected with replication-defective, full length, artificial retroviruses containing the ALB/VZV TK chimaera (pCR74) compared to parental cell lines which were not infected. This demonstrates the selectivity of expression for VZV TK in hepatoma verse non-hepatoma cells.

### LEGEND TO FIGURES

- Figure 1: : Schematic representation of albumin enhancer and promoter sequences in relation to the albumin gene (IA), a heterologous gene (IB) and heterologous gene without non-essential sequences.
- Figure 2: : Diagram of Varicella Zoster Thymidine Kinase Gene.
- Figure 2B: : VZV TK gene - 1^{o} sequence.
- Figure 3A: : Albumin transcriptional regulatory sequence / VZV TK molecular chimaera.
- Figure 3B: : Alpha-fetoprotein transcriptional regulatory sequence / VZV TK molecular chimaera.
- Figure 4: : Proviral form of retrovirus containing alpha-fetoprotein / VZV TK molecular chimaera.
- Figure 4B: : pCR78.
- Figure 5: : Flow chart showing the construction of pCR74.
- Figure 6: : Flow chart showing the construction of pCR78.
- Figure 7: : Sequence flanking ALB E/P to VZV TK in pCR74.
- Figure 8: : Sequence flanking AFP E/P to VZV TK in pCR78.
- Figure 9: : Production of ara ATP with cells infected with controls, pCR74, pCR78.

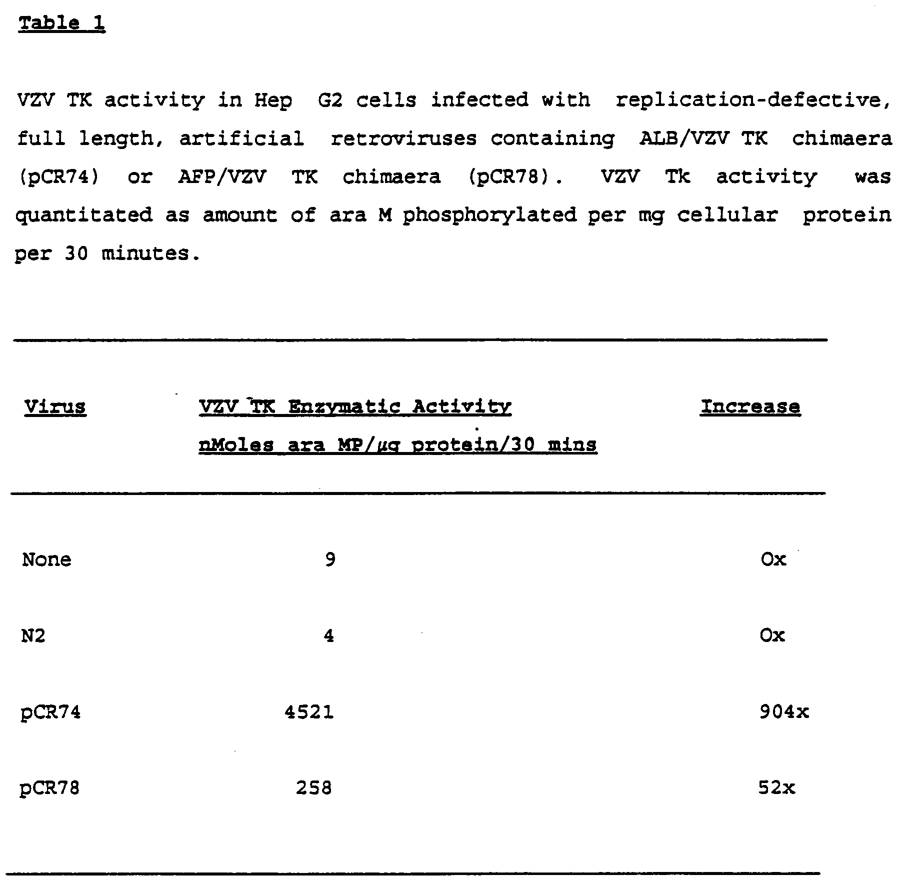

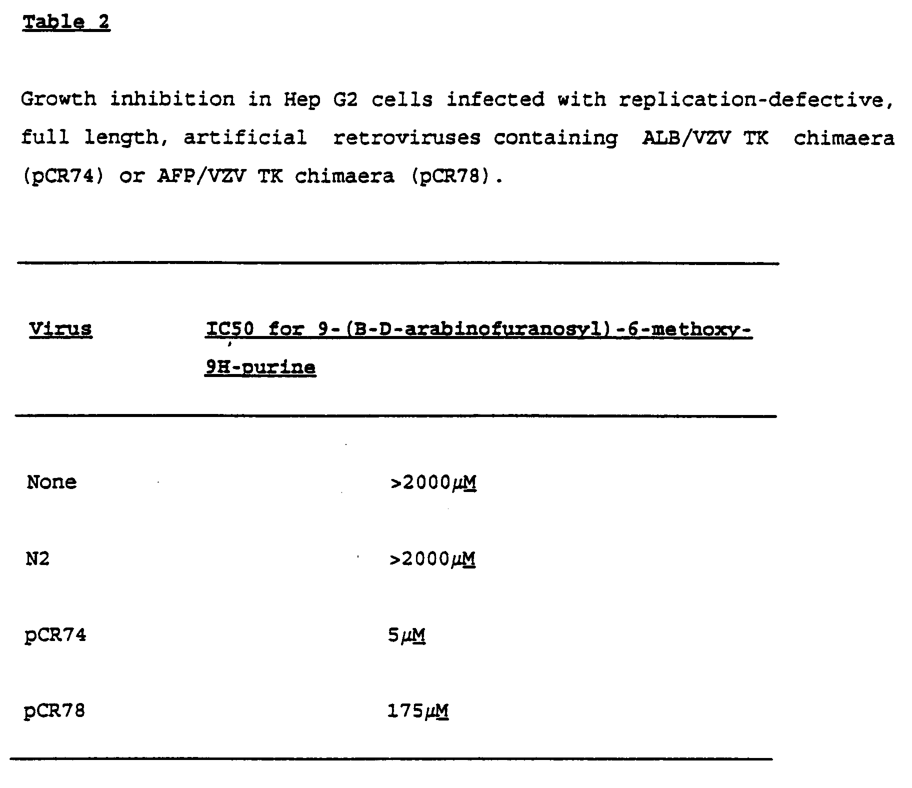

## Claims

1. Use of a molecular chimaera for the manufacture of a medicament for cancer therapy by a method which comprises administering the molecular chimaera and a prodrug to a patient, the molecular chimaera comprising a transcriptional regulatory DNA sequence capable of selective activation in a mammalian cancer cell and a DNA sequence operatively linked to the transcriptional regulatory DNA sequence and encoding a heterologous enzyme capable of catalysing the conversion of the prodrug into an agent toxic to the cancer cell.

2. Use of a prodrug for the manufacture of a medicament for cancer therapy by a method which comprises administering the prodrug and a molecular chimaera to a patient, the molecular chimaera comprising a transcriptional regulatory DNA sequence capable of selective activation in a mammalian cancer cell and a DNA sequence operatively linked to the transcriptional regulatory DNA sequence and encoding a heterologous enzyme capable of catalysing the conversion of the prodrug into an agent toxic to the cancer cell.

3. Use according to either of claims 1 and 2, wherein the method involves administration of the molecular chimaera by calcium phosphate transfection, electroporation, microinjection, liposomal transfer, ballistic barrage or retroviral infection.

4. Use according to any of claims 1 to 3, wherein the heterologous enzyme that is capable of catalysing the conversion of the prodrug into an agent toxic to the cancer cell is carboxypeptidase G2.

5. Use according to claim 4, wherein the prodrug is para-N-bis(2-chloroethyl)aminobenzoyl glutamic acid.

6. Use according to any of claims 1 to 3, wherein the heterologous enzyme that is capable of catalysing the conversion of the prodrug into an agent toxic to the cancer cell is alkaline phosphatase.

7. Use according to claim 6, wherein the prodrug is etoposidephosphate, doxorubicin phosphate or mitomycin phosphate.

8. Use according to any of claims 1 to 3, wherein the heterologous enzyme that is capable of catalysing the conversion of the prodrug into an agent toxic to the cancer cell is penicillin-V-amidase.

9. Use according to claim 8, wherein the prodrug is a phenoxyacetamide derivative of doxorubicin or melphalan.

10. Use according to any of claims 1 to 3, wherein the heterologous enzyme that is capable of catalysing the conversion of the prodrug into an agent toxic to the cancer cell is cytosine deaminase.

11. Use according to claim 10, wherein the prodrug is 5-fluorocytosine.

12. Use according to any of claims 1 to 3, wherein the heterologous enzyme that is capable of catalysing the conversion of the prodrug into an agent toxic to the cancer cell is varicella zoster virus thymidine kinase.

13. Use according to claim 12, wherein the prodrug is a purine or pyrimidine arabinoside.

14. Use according to claim 13, wherein the prodrug is 9-β-arabinofaranosyl-6-methoxy-9H-purine or 1-(β-d-arabinofuranosyl)-5-propynluracil.
